# EUROPEAN PATENT SPECIFICATION

(11) **EP 0 545 911 B1**
(45) Date of publication and mention of the grant of the patent: **11.09.1996**
(21) Application number: 93200152.2
(22) Date of filing: 16.06.1989
(51) Int. Cl.: A61K 38/00

(54) **Lanthionine-containing bacteriocin compositions for use as bactericides**
Arzneimittel, die Lanthionin enthaltenden Bacteriozin enthalten, zur Verwendung als Bakterizide
Compositions de bactériocin à lanthionine utilisées en tant que bactéricides

(30) Priority: 01.03.1989 US 317626; 22.06.1988 US 209861
(43) Date of publication of application: 09.06.1993
(62) Divisional of application: 89907595.6
(73) Proprietor: APPLIED MICROBIOLOGY, INC., Brooklyn, N.Y. 11232 (US)
(72) Inventor: Blackburn, Peter, New York, New York 10036 (US); Polak, June, Brooklyn, New York 11201 (US); Gusik, Sara-Ann, New York, New York 10003 (US); Rubino, Stephen D., Berkeley Heights, New Jersey 07922 (US)
(74) Representative: Lucas, Brian Ronald

(56) References cited:
- CHEMICAL ABSTRACTS, vol. 83, no. 15, 14 May 1975, Columbus, Ohio, US; abstract no. 126868, FINN, V.G. 'Effect of surfactants on the sensitivity of microbial cells to bactericins'
- CHEMICAL ABSTRACTS, vol. 86, no. 1, 3 January 1977, Columbus, Ohio, US; abstract no. 594, REDENKO, A.I. ET AL. 'Effect of the antibiotic nisin on pathogenic staphylococci and steptococci'

## Description

Nisin is a polypeptide with antimicrobial properties which is produced in nature by various strains of the bacterium Streptococcus lactis. It is a known food preservative which inhibits the outgrowth of spores of certain species of Gram positive Bacilli.

Although sometimes mistakenly and imprecisely referred to as an antibiotic, nisin is more correctly classified as a bacteriocin, i.e., a proteinaceous substance produced by bacteria and which has antibacterial activity only towards species closely related to the species of its origin. Nisin is a naturally-occurring preservative found in low concentration in milk and cheese, and is believed to be completely non-toxic and non-allergenic to humans.

Nisin has recently been recognized as safe by the FDA as a direct food ingredient in pasteurized cheese spread, pasteurized processed cheese spread, and pasteurized or pasteurized processed cheese spread with fruits, vegetables, or meats. Furthermore, since it is a polypeptide, any nisin residues remaining in foods are quickly digested.

A summary of nisin's properties appears in Hurst, A., Advances in Applied Microbiology 27:85-123 (1981). This publication describes what is generally known about nisin. Nisin, produced by Streptococcus lactis, is available commercially as an impure preparation, Nisaplin™, from Aplin & Barrett Ltd., Dorset, England and can be obtained by isolating naturally-occurring nisin from cultures of Streptococcus lactis and then concentrating the nisin according to known methods. There are also reported methods for producing nisin using altered strains of Streptococcus. See Gonzalez et al., US Pat. No. 4,716,115, issued December 29, 1987. It should also be possible to produce nisin by recombinant DNA technology.

Nisin has been applied effectively as a preservative in dairy products, such as processed cheese, cream and milk. The use of nisin in processed cheese products has been the subject of recent patents. See US Pat. Nos. 4,584,199 and 4,597,972. The use of nisin to inhibit the growth of certain Gram positive bacteria has been well documented. However, its complete success and acceptance as a food preservative has heretofore been hampered by the belief that nisin was ineffective against Gram negative and many Gram positive bacteria. Gram negative bacteria are almost always present in conjunction with Gram positive bacteria and are a major source of food spoilage and contamination. See Taylor, US Pat. No. 5,584,199, issued April 22, 1986 and Taylor, US Pat. No. 4,597,972, issued July 1, 1986; Tsai and Sandine, "Conjugal Transfer of Nisin Plasmid Genes from Streptococcus Lactis 7962 to Leuconostoc Dextranicum 181, Applied and Environmental Microbiology, Feb. 1987, p. 352; "A Natural Preservative", Food Engineering Int'l, May 1987, pp. 37-38; "Focus on Nisin", Food Manufacture, March 1987, p. 63.

It has now been found that in the presence of surfactant alone, nisin has enhanced activity against Gram positive bacteria.

The parent application relates to compositions of enhanced bactericidal activity comprising a lanthionine-containing bacteriocin and a chelating agent. These compositions can also contain a surfactant.

It has now been found that in the presence of surfactant alone, nisin has enhanced activity against Gram positive bacteria.

This invention provides bacteriocin compositions of nisin or other, lanthionine containing bacteriocins, in combination with surfactants. The invention further provides the compositions dissolved or suspended in a suitable carrier to yield enhanced broad range bactericides.

In the present invention, surfactants, valuable as cleansing agents, suitable for combination with nisin include, but are not limited to, the nonionic surfactants Tweens, Tritons, and glycerides, ionic surfactants such as fatty acids, quaternary compounds, anionic surfactants and amphoteric surfactants such as cocamidopropyl betaine and emulsifiers.

Since Gram positive and Gram negative bacteria are almost always found together in foods, the effectiveness of the nisin compositions towards Gram negative bacteria such as Salmonella typhimurium, Escherichia Coli, Klebsiella pneumoniae, Pseudomonas aeruginosa, Bacterioides gingivalis, Actinobacillus actinomycetescomitans, and other Gram negative pathogens and Gram positive bacteria will be of great use. The bactericides are particularly suited for the control and prevention of contamination of raw ingredients, processed foods and beverages by bacterial pathogens and other microbial spoilage organisms. Potential food related uses include treatment of meats, especially poultry, eggs, cheese and fish and treatment of food packaging and handling equipment. Further uses include as food preservative, such as in processed cheese, cream, milk, dairy products and in cleaning poultry, fish, meats, vegetables, and dairy and food processing equipment. The use of the nisin compositions should not be limited to food related used and the nisin compositions should be useful in any situation in which there is a need or desire to eliminate Gram negative and Gram positive bacteria.

The compositions can be dissolved in a suitable carrier for example an aqueous solvent or buffer or suspended in any suitable liquid, colloidal or polymeric matrix to create bactericides. Such compositions preferably contain from 0.01 to 0.2 percent of the surfactant. The compositions or bactericides can be incorporated into ointments or coatings for medicinal uses such as the treatment of infections, wound dressings or surgical implants and as a broad spectrum disinfectant for skin or oral rinses, disinfectant scrubs, wipes or lotions. The bactericides can be used for cleaning medical instruments, in pre-operative surgical scrubs and the like. The bactericides are particularly useful in circumstances where environmental disinfection is desired but where chemical germicidals are precluded because of the risks of corrosive or otherwise toxic residues.

Unlike the activity of most broad spectrum germicidals which is compromised by the presence of complex organic matter, the compositions of the present invention are effective as bactericides in the presence of organic matter, such as milk or serum.

Nisin belongs to the class of peptide bacteriocins containing lanthionine. Also included among that class are subtilin, epidermin, cinnamycin, duramycin, ancovenin and Pep 5. These bacteriocin peptides are each produced by different microorganisms. However, subtilin obtained from certain cultures of Bacillus subtilis, and epidermin obtained from certain cultures of Staphylococcus epidermidis, have been found to have molecular structures very similar to that of nisin (see Hurst, pp. 85-86, and Schnell et al., Nature, 333:276-278). It is therefore believed that because of the molecular similarities, other lanthionine-containing peptide bacteriocins will be equally as effective as nisin in combination with non-ionic surfactants in eliminating Gram negative and Gram positive bacterial contaminations.

The effectiveness of the nisin, and by extension other lanthionine-containing peptide bacteriocin, compositions as bactericides against Gram negative bacteria is surprising, since the prior art generally teaches away from this activity of nisin.

In order to demonstrate the superior and unexpected rapid activity of the composition containing nisin and various surfactants against Gram positive bacteria, a number of experiments were conducted with the bactericides. These experiments are meant as illustration and are not intended to limit this invention. It is to be expected that other, lanthionine-containing peptide bacteriocins would be effective substitutes for nisin.

All tests in the following examples were performed at 37°C. The efficacy of the enhanced broad range bactericides was determined by assaying bactericidal activity as measured by the percent bacterial survival after treatment with the bactericide. Generally, after incubation of a 10⁷ cell per ml suspension of target species with the novel bactericide for specified lengths of time, bacteria were collected by centrifugation for 2 minutes. The bacterial pellet was washed free of the bactericide with a rescue buffer, termed herein Phage buffer (50mM Tris-HCl buffer pH 7.8, 1mM MgSO₄, 4mM CaCl₂, 0.1 M NaCl, and 0.1% gelatin), resuspended and serially diluted into Phage buffer, and 100ml of the suspended bacteria were spread on nutrient agar plates. Surviving bacteria were determined by scoring colony forming units (CFU) after incubation for 24-48 hours at 37°C. An effective bactericide according to this invention is one which allows less than 0.1% of the initial viable count of the bacteria to survive.

### Example 1

### Nisin and Surfactant (monolaurin) Activity Against Gram Positive Bacteria

The bactericidal activity of nisin can be significantly enhanced when combined with a surfactant alone. This is best illustrated at a limiting nisin concentration (0.2 µg/ml) as shown in Table A. At concentrations up to 0.1%, the food grade surfactant monolaurin has little significant bactericidal activity towards Streptococcus agalactiae in the complex medium milk. Nisin, at concentrations up to 0.2 µg.ml, likewise does not exhibit significant bactericidal activity in milk. However, the combination of the two agents, 0.1% monolaurin and nisin 0.2 g/ml, is extremely potent towards S. agalactiae. This bactericide is over 100 times more active than what would be expected for the additive effect and 10,000 times more active than either of the components individually. Thus, when the application of nisin is limited by its available activity, a bactericide comprising nisin with a surfactant can be expected to be more useful.

| Nisin Bactericidal Activity towards Streptococcus agalactiae in milk at 37°C (Activation of nisin by monolaurin) | | | |
|---|---|---|---|
| Nisin (µ g/ml) | Monolaurin (%) | | |
| | 0 | 0.01 | 0.1 |
| | % survival at 2hr^{a} | | |
| 0 | 100 | 100 | 4.5 |
| 0.02 | 100 | 100 | 0.2 |
| 0.2 | 2.2 | 0.05 | 0.0008 |

| | | | |
|---|---|---|---|
| ^{a} Initial viable counts 6.0 x 10⁷ cfu/ml. Incubations were in milk at 37°C. | | | |

### Example 2

### Nisin and Surfactant (glyceride monooleate) Activity Against Gram positive Bacteria

An example of where the application of nisin is limited by its available activity is illustrated by the data in Table B. Although nisin is bactericidal towards L. monocytogenes, the data in Table B demonstrate that in a complex medium like milk the available nisin activity towards this organism is restricted. However, the bactericide comprised of nisin with the glyceride monooleate is effective in milk towards this foodborne pathogen, even though monooleate by itself had no bactericidal activity towards this organism.

**Table B**

| Nisin Bactericidal Activity towards Listeria monocytogenes in milk at 37°C (Activation of nisin by monooleate) | | | |
|---|---|---|---|
| Nisin (µ g/ml) | % Monooleate | | |
| | 0 | 0.1 | 1.0 |
| | % survival at 2hr^{a} | | |
| 0 | 100 | 67 | 63 |
| 100 | 0.56 | 10⁻³ | 10⁻⁴ |

| | | | |
|---|---|---|---|
| ^{a} Initial viable count 5.0 x 10⁷ cfu/ml. Incubations were in milk at 37°C. | | | |

## Claims

1. A bactericidal composition comprising a lanthionine-containing bacteriocin, characterized in that it includes a surfactant, but excludes a chelating agent.

2. A composition according to Claim 1, characterized in that the surfactant is non-ionic.

3. A composition according to Claim 1, characterized in that the surfactant is monolaurin or glyceride monooleate.

4. A composition according to Claim 1, characterized in that the surfactant is selected from the group consisting of Tritons, Tweens, glycerides, fatty acids, emulsifiers, quaternary compounds, amphoteric and anionic surfactants.

5. A composition according to any preceding Claim, characterized in that the lanthionine-containing bacteriocin is nisin or subtilin.

6. A composition according to any preceding Claim, characterized in that it further comprises a carrier.

7. A composition according to Claim 6, characterized in that the concentration of surfactant is from 0.01 to 1.0 percent.

8. A composition according to Claim 6 or 7, characterized in that the carrier is an aqueous solvent or buffer.

9. Use, other than in surgery or therapy, in combination of (1) a lanthionine-containing bacteriocin and (2) a surfactant, in the absence of a chelating agent, as a bactericide effective against Gram positive bacteria.

10. Use according to Claim 9 for preserving food.

11. Use according to Claim 10 for preserving dairy products.

12. Use according to Claim 11 for preserving milk.

13. Use according to Claim 9 for cleaning poultry, fish, meats vegetables and dairy and food processing equipment.

14. A composition according to any one of Claims 1 to 8 for medicinal use.

15. A composition according to Claim 14 for use in the treatment of infections, wound dressings or surgical implants.

16. A composition according to Claim 14 for use as a broad spectrum disinfectant for skin or oral rinses, disinfectant scrubs, wipes or lotions.

17. An enhanced broad range bactericide comprising a carrier, a lanthionine-containing bacteriocin and a surfactant, but not containing a chelating agent.

18. A bactericide according to Claim 17, wherein the surfactant is selected from the group consisting of Tritons, Tweens, glycerides, fatty acids, emulsifiers, quaternary compounds, amphoteric and anionic surfactants and is present in an amount sufficient such that the bactericide has enhanced effectiveness against at least one of the bacteria from the group consisting of Gram negative and Gram positive bacteria.

19. A bactericide according to Claim 18, wherein the concentration of surfactant is between about 0.01 and 1.0 percent.

## Patentansprüche

1. Bakterizide Zusammensetzung bestehend aus einem Lanthionin enthaltenden Bakteriocin, dadurch gekennzeichnet, daß sie ein Tensid enthält, aber einen Chelatbildner ausschließt.

2. Zusammensetzung nach Anspruch 1, dadurch gekennzeichnet, daß das Tensid nicht-ionisch ist.

3. Zusammensetzung nach Anspruch 1, dadurch gekennzeichnet, daß das Tensid Monolaurin oder Glycerid-Monooleat ist.

4. Zusammensetzung nach Anspruch 1, dadurch gekennzeichnet, daß das Tensid aus der Gruppe bestehend aus Tritonen, Tweens, Glyceriden, Fettsäuren, Emulgatoren, quartären Verbindungen, amphotären und anionischen Tensiden ausgewählt ist.

5. Zusammensetzung nach einem der vorhergehenden Ansprüche, dadurch gekennzeichnet, daß das Lanthionin enthaltende Bakteriocin Nisin oder Subtilin ist.

6. Zusammensetzung nach einem der vorhergehenden Ansprüche, dadurch gekennzeichnet, daß sie ferner einen Träger enthält.

7. Zusammensetzung nach Anspruch 6, dadurch gekennzeichnet, daß die Konzentration des Tensids 0,01 bis 1,0 Prozent beträgt.

8. Zusammensetzung nach Anspruch 6 oder 7, dadurch gekennzeichnet, daß der Träger ein wäßriges Lösemittel oder ein wäßriger Puffer ist.

9. Verwendung, anders als in der Chirurgie oder Therapie, in Kombination von (1), einem Lanthionin enthaltenden Bakteriocin und (2), einem Tensid, in Abwesenheit eines Chelatbildners, als ein Bakterizid, wirksam gegen Gram-positive Bakterien.

10. Verwendung nach Anspruch 9 zum Schutz von Lebensmitteln.

11. Verwendung nach Anspruch 10 zum Schutz von Molkereiprodukten.

12. Verwendung nach Anspruch 11 zum Schutz von Milch.

13. Verwendung nach Anspruch 9 zur Reinigung von Geflügel, Fisch, Fleisch, Gemüse, Molkerei- und Lebensmittelausrüstungsgegenständen.

14. Zusammensetzung nach einem der Ansprüche 1 bis 8 zur medizinischen Verwendung.

15. Zusammensetzung nach Anspruch 14 zur Verwendung bei der Behandlung von Infektionen, Wundabdeckungen oder chirurgischen Implantaten.

16. Zusammensetzung nach Anspruch 14 zur Verwendung als Breitspektrum-Desinfektionsmittel für die Haut oder als Mundspülmittel, Desinfektionsreinigungsmittel, zum Abwischen oder als Lotion.

17. Verstärktes Breitbandbakterizid, bestehend aus einem Träger, einem Lanthionin enthaltenden Bakteriocin und einem Tensid, aber ohne einen Chelatbildner.

18. Bakterizid nach Anspruch 17, bei dem das Tensid aus der Gruppe bestehend aus Tritonen, Tweens, Glyceriden, Fettsäuren, Emulgatoren, quartären Verbindungen, amphotären und anionischen Tensiden ausgewählt ist und in einer zufriedenstellenden Menge vorhanden ist, so daß das Bakterizid eine verstärkte Wirkung gegen Bakterien aus der Gruppe bestehend aus Gram-negativen und Gram-positiven Bakterien hat.

19. Baktericid nach Anspruch 18, wobei die Konzentration des Tensids zwischen 0,01 und 1,0 Prozent liegt.

## Revendications

1. Composition bactéricide comprenant une bactériocine contenant de la lanthionine, caractérisée en ce qu'elle comprend un agent tensio-actif, mais qu'elle exclue un agent chélatant.

2. Composition selon la revendication 1, caractérisée en ce que l'agent tensio-actif est non ionique.

3. Composition selon la revendication 1, caractérisée en ce que l'agent tensio-actif est de la monolaurine ou un monooléate de glycéride.

4. Composition selon la revendication 1, caractérisée en ce que l'agent tensio-actif est choisi dans le groupe constitué par les Tritons, les Tweens, les glycérides, les acides gras, les émulsifiants, les composés quaternaires, les agents tensio-actifs amphotères et anioniques.

5. Composition selon l'une des revendications précédentes, caractérisée en ce que la bactériocine contenant de la lanthionine est la nisine ou la subtiline.

6. Composition selon l'une des revendications précédentes, caractérisée en ce qu'elle comporte en outre un véhicule.

7. Composition selon la revendication 6, caractérisée en ce que la concentration en agent tensio-actif est de 0,01 à 1,0 %.

8. Composition selon la revendication 6 ou 7, caractérisée en ce que le véhicule est un solvant ou un tampon aqueux.

9. Utilisation, autre qu'en chirurgie ou thérapie, en combinaison, de (1) une bactériocine contenant de la lanthionine et (2) un agent tensio-actif, en l'absence d'agent chélatant, comme bactéricide efficace contre les bactéries à Gram positif.

10. Utilisation selon la revendication 9 pour conserver les aliments.

11. Utilisation selon la revendication 10 pour conserver les produits laitiers.

12. Utilisation selon la revendication 11, pour conserver le lait.

13. Utilisation selon la revendication 9, pour nettoyer la volaille, le poisson, les viandes, les légumes, les équipements de traitements de produits laitiers et alimentaires.

14. Compositions selon l'une quelconque des revendications 1 à 8, pour une utilisation médicinale.

15. Composition selon la revendication 14, pour utilisation dans le traitement d'infections, de pansements de blessures ou d'implants chirurgicaux.

16. Composition selon la revendication 14 pour utilisation comme désinfectant à large spectre pour les rinçages de la peau ou bucaux, les brosses, mouchoirs ou lotions désinfectantes.

17. Bactéricide à large spectre amélioré comprenant un véhicule, une bactériocine contenant de la lanthionine et un agent tensio-actif mais ne contenant pas d'agent chélatant.

18. Bactéricide selon la revendication 17, dans lequel l'agent tensio-actif est choisi parmi le groupe constitué par les Tritons, les Tweens, les glycérides, les acides gras, les émulsifiants, les composants quaternaires, les agents tensio-actifs amphotères et anioniques et est présent en une quantité suffisante pour que le bactéricide ait une efficacité améliorée contre au moins une des bactéries du groupe constitué par les bactéries à Gram négatif et à Gram positif.

19. Bactéricide selon la revendication 18, dans lequel la concentration en agent tensio-actif est comprise entre environ 0.01 et 1.0 %.
